# EUROPÄISCHE PATENTANMELDUNG

(11) **EP 4 534 043 A1**
(43) Veröffentlichungstag der Anmeldung: **09.04.2025**
(21) Anmeldenummer: 24204280.2
(22) Anmeldetag: 02.10.2024
(51) Int. Cl.: A61D 7/00, A61D 1/02, A61J 7/00, A61M 5/00

(54) **VORRICHTUNG ZUM EINFÜHREN EINES MITTELS IN WENIGSTENS EIN NUTZTIER**

(30) Priorität: 04.10.2023 DE 102023126974
(71) Anmelder: Gerdes, Gerd, 49681 Garrel (DE)
(72) Erfinder: Gerdes, Gerd, 49681 Garrel (DE)
(74) Vertreter: Jabbusch, Matthias

(57) **Zusammenfassung**

Bei einer Vorrichtung zum Einführen eines Mittels, insbesondere eines Arzneimittels, in wenigstens ein Nutztier, ist vorgesehen, dass sie nach Art einer Spritze mit Kolben und Zylinder ausgebildet ist, wobei der Zylinder an seinem dem Einführen des Kolbens in den Zylinder entgegengesetzten Ende mit zumindest einer Öffnung ausgerüstet ist.

Mit dieser Vorrichtung gelingt eine möglichst vollständige Einführung insbesondere eines Arzneimittels insbesondere in das Maul eines Nutztieres.

## Beschreibung

Die Erfindung betrifft eine Vorrichtung zum Einführen eines Mittels, insbesondere eines Arzneimittels, in wenigstens ein Nutztier.

Nutztiere, wie Kühe oder Pferde, können erkranken, zur Heilung der Tiere werden dann Arzneimittel eingesetzt, diese sind den Tieren zuzuführen bzw. in diese einzuführen.

So ist es bekannt, flüssige Arzneimittel oder aufgelöste Arzneimittel mit Hilfe von Spritzen z.B. in die Muskulatur eines Nutztieres einzuführen. Dies ist aufwendig und benötigt die Zuarbeit eines Tierarztes. Daher wurde im Stand der Technik bereits vorgeschlagen, Arzneimittel für Tiere, insbesondere für Nutztiere, mit dem Futter den Tieren zuzuführen. So werden beispielsweise in Tablettenform vorliegende Arzneimittel in eine Pulverform überführt, die dann ins Futter gemischt wird. Ggf. wird ein derartiges pulvriges Arzneimittel auch auf das Futter aufgestreut.

Es hat sich aber gezeigt, dass Nutztiere, insbesondere Pferde, das Futter zu sich nehmen, dabei aber das Arzneimittel verschmähen und weitgehend liegen lassen. Gerade Pferde sind eitel und fressen ihnen unbekannte z.B. weiße Bestandteile nicht. Es wurde aber beobachtet, dass ein Medikament für ein Fohlen von der Stute gefressen wurde. Wurmkur-Medikamente in Pastenform bleiben am Gaumen der Tiere kleben. Und die schon genannte Auflösung von Tabletten oder Pulvern führt bei schlechter und/oder vollständiger Auflösung zur Ausbildung eines Bodensatzes und somit zu unvollständiger Medikation des Nutztieres.

Der Erfindung liegt die Aufgabe zugrunde, eine Vorrichtung der eingangsgenannten Gattung aufzuzeigen, mit der eine möglichst vollständige Einführung insbesondere eines Arzneimittels gelingt.

Diese Aufgabe ist erfindungsgemäß dadurch gelöst, dass die Vorrichtung nach Art einer Spritze mit Kolben und Zylinder ausgebildet ist, wobei der Zylinder an seinem dem Einführen des Kolbens in den Zylinder entgegengesetzten Ende mit zumindest einer Öffnung ausgerüstet ist.

Die erfindungsgemäße Vorrichtung nutzt eine Spritze, wie sie beispielsweise in Human- und Veterinärmedizin insbesondere in Einwegform vielfach vorliegt. Diese Spritze hat eine Umgestaltung darin erfahren, dass der Zylinder der Spritze an seinem Ende, das dem Einführen des Kolbens in den Zylinder gegenüberliegt, nicht mit einer Verengung oder einer Aufsatzmöglichkeit für eine Kanüle ausgebildet ist. Vielmehr weist die Spritze an einem Ende des Kolbens eine Öffnung auf.

Über diese Öffnung ist es möglich, die Spritze mit pulverförmigen oder auch tablettenförmigen Arzneimitteln oder anderen Mitteln zu füllen und diese anschließend durch Eindrücken des Kolbens in den Zylinder in das geöffnete oder offengehaltene Maul eines Nutztieres einzuführen.

Die erfindungsgemäße Vorrichtung stellt also eine einfache Applizierungsvorrichtung für Arzneimittel direkt in das Maul eines Tieres bereit. Die Zylinderform der Spritze bewirkt ein zielgerichtetes Einwerfen der Arzneimittel, eine nahezu vollständige Aufnahme durch das Nutztier ist damit vorteilhaft gewährleistet.

Nach einer ersten Weiterbildung der Erfindung ist vorgesehen, dass die Öffnung am vorzugsweisen freien Ende des Zylinders den ganzen Querschnitt des Zylinders abdeckt. Der Zylinder kann an dem Einführen des Kolbens in den Zylinder entgegen gelegenen Ende einfach abgeschnitten sein, so dass sich die Öffnung über den gesamten Querschnitt des Zylinders erstreckt.

Eine nächste Weiterbildung der Erfindung sieht vor, dass die Länge des Kolbens länger ist als die Länge des Zylinders. Der in den Zylinder eingeführte und im weiteren Verlauf des Zylinders geführte Kolben kann mit seinem regelmäßig mit einer Schiebefläche ausgerüsteten Ende bis aus dem freien Ende des Zylinders herausgeführt werden. Dabei ergibt sich beim Einführen von Arzneimittelpulvern noch ein Katapulteffekt, da der Kolben an einem nach Innen angerichteten Grat der Öffnung des Zylinders noch aufgehalten werden kann und erst durch weitere Druckausübung beschleunigt aus dem Inneren des Zylinders hervortritt. Die so erzielte Katapultwirkung bewirkt eine Beschleunigung beim Einführen der Arzneimittel in das Maul eines Nutztieres.

Schließlich kann noch vorgesehen sein, dass Kolben und Zylinder jeweils aus Kunststoff gefertigt sind, beispielsweise in Form einer Einwegspritze.

Ein Ausführungsbeispiel der Erfindung ist in der Zeichnung dargestellt. Es zeigen:
- Figur 1:: eine Seitenansicht der erfindungsgemäßen Vorrichtung zum Einführen eines Arzneimittels;
- Figur 2:: eine weitere Seitenansicht der Vorrichtung gemäß Figur 1 und
- Figur 3:: eine weitere Seitenansicht der Vorrichtung gemäß Figur 1 unter Vereinzelung ihrer Bauteile.

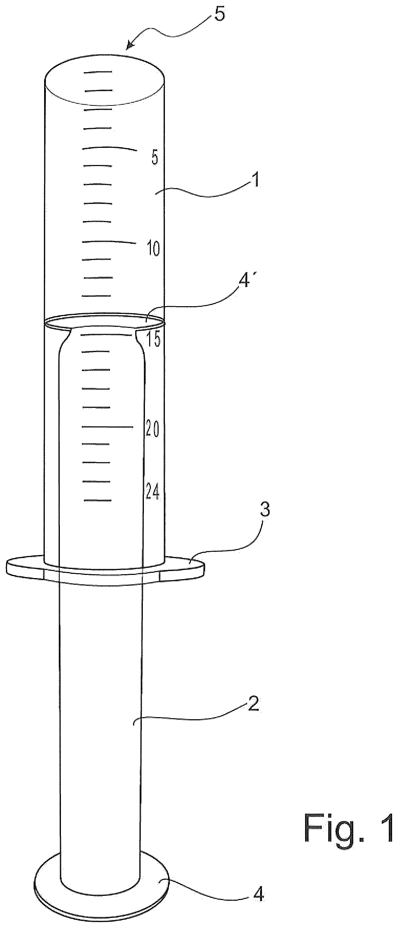

Die Vorrichtung in Figur 1 ist nach Art einer Spritze ausgebildet. Sie weist einen Zylinder 1 und einen Kolben 2 auf. Der Zylinder 1 hat an einem Ende Verbreiterungen 3 zur Auflage von Fingern, während der Kolben 2 an beiden Seiten scheibenförmige Verbreiterungen 4, 4' aufweist. Während die Verbreiterung 4 zum Auflegen eines Daumens bei Benutzung der Spritze dient, füllt die Verbreiterung 4` den Innenquerschnitt des Zylinders 1 aus.

Das dem Einführen des Kolbens 2 in den Zylinder 1 abgekehrte freie Ende des Zylinders 1 ist mit einer Öffnung 5 ausgerüstet. Der Zylinder 1 ist zum Ausbilden der Öffnung 5 abgeschnitten, die Größe der Öffnung 5 entspricht dem Innenquerschnitt des Zylinders 1.

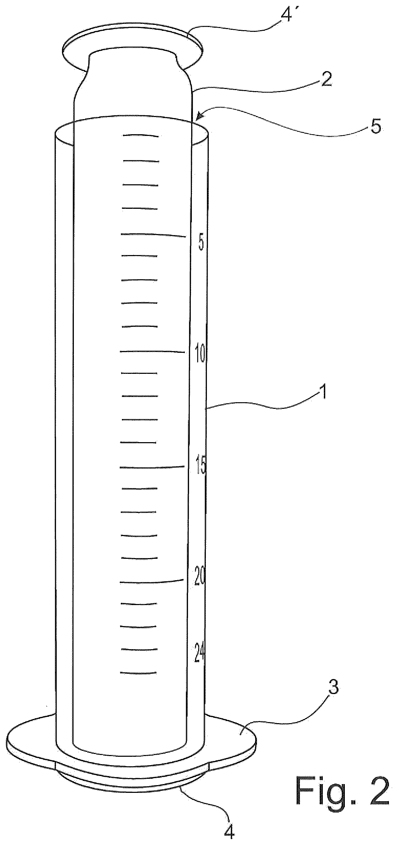

In Figur 2 ist der Kolben 2 maximal weit in den Zylinder 1 eingeschoben, so dass die Verbreiterung 4` des Kolbens 2 über die Öffnung 5 des Zylinders 1 hinaus vorgeschoben ist. Die Länge des Kolbens 2 ist größer als die Länge des Zylinders 1.

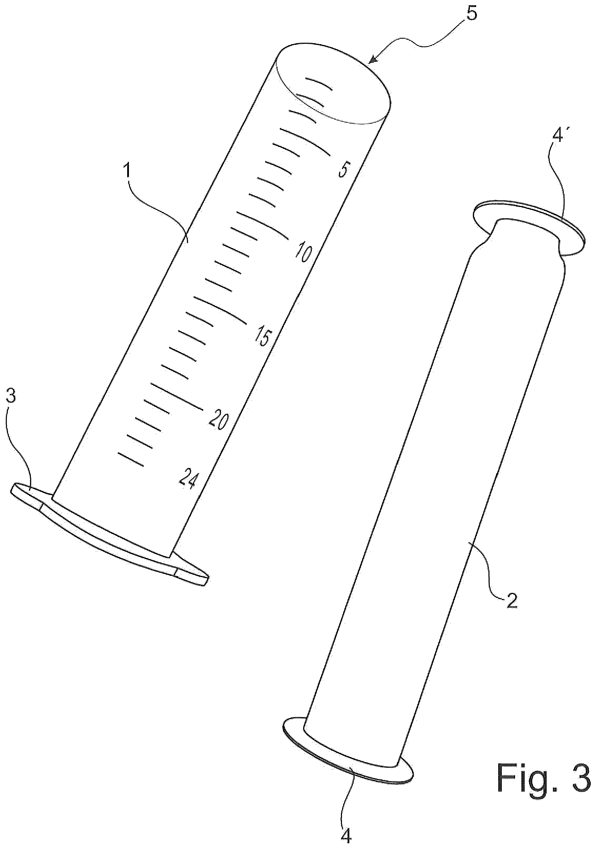

Diese verschiedenen Längenverhältnisse von Kolben 2 und Zylinder 1 sind auch in Figur 3 zu erkennen. Hier ist noch einmal deutlich die Öffnung 5 des Zylinders 1 dargestellt.

## Patentansprüche

1. Vorrichtung zum Einführen eines Mittels, insbesondere eines Arzneimittels, in wenigstens ein Nutztier,
**dadurch gekennzeichnet,**
**dass** sie nach Art einer Spritze mit Kolben (2) und Zylinder (1) ausgebildet ist, wobei der Zylinder (1) an seinem dem Einführen des Kolbens (2) in den Zylinder (1) entgegengesetzten Ende mit zumindest einer Öffnung (5) ausgerüstet ist.

2. Vorrichtung nach Anspruch 1, **dadurch gekennzeichnet, dass** die Öffnung (5) am Ende des Zylinders (1) den ganzen Querschnitt des Zylinders (1) abdeckt.

3. Vorrichtung nach Anspruch 1 oder 2, **dadurch gekennzeichnet, dass** die Öffnung (5) mit einem nach Innen angerichteten Grat ausgerüstet ist.

4. Vorrichtung nach einem der vorhergehenden Ansprüche, **dadurch gekennzeichnet, dass** die Länge des Kolbens (2) größer als die Länge des Zylinders (1) ist.

5. Vorrichtung nach einem der vorhergehenden Ansprüche, **dadurch gekennzeichnet, dass** Kolben (2) und Zylinder (1) jeweils aus Kunststoff gefertigt sind.
